Europäisches Patentamt

(19) European Patent Office

Office européen des brevets

(11) Publication number: **0 169 650**
A2

## (12) .EUROPEAN PATENT APPLICATION

(21) Application number: 85304225.7

(51) Int. Cl.⁴: **C 07 C 125/06**

(22) Date of filing: 13.06.85

(30) Priority: 14.06.84 IT 2140184

(43) Date of publication of application:
29.01.86 Bulletin 86/5

(84) Designated Contracting States:
BE CH DE FR GB LI NL

(71) Applicant: Montedison S.p.A.
31, Foro Buonaparte
I-20121 Milan(IT)

(72) Inventor: Alessio, Enzo
12, via Alfieri
I-34170 Gorizia(IT)

(72) Inventor: Mestroni, Giovanni
27, Vicolo Castagneto
I-34127 Trieste(IT)

(74) Representative: Whalley, Kevin et al,
Marks & Clerk 57/60 Lincoln's Inn Fields
London WC2A 3LS(GB)

(54) Process for preparing aromatic urethanes from nitroaromatic compounds.

(57) A process for preparing aromatic urethanes by reacting a nitroaromatic compound $Ar(NO_2)_x$ with carbon monoxide and an alcohol at a temperature of from 25° to 250°C and at a pressure of from 1 to 150 atm, in the presence of a catalyst consisting of (1) a noble metal supported on an inert carrier and of an aromatic bidentate nitrogenous helating agent (Chel), or of a salt of a noble metal $(MY_2)$ and of said chelating agent, which may be optionally combined as a complex of formula $M(Chel)Y_2$, and optionally (2) of a Brönsted acid, wherein Ar is an aryl or a heteroaryl radical, optionally substituted with one or more groups inert under the reaction conditions, x equals 1, 2 or 3, M is a noble metal, and Y is an organic or inorganic cation.

EP 0 169 650 A2

Croydon Printing Company Ltd.

"PROCESS FOR PREPARING

AROMATIC URETHANES

FROM

NITROAROMATIC COMPOUNDS"

This invention relates to a process for preparing aromatic urethanes from nitroaromatic compounds, and in particular by catalytic reductive carbonylation by means of carbon monoxide of nitroaromatic compounds in the presence of an alcohol, using a particular catalytic system.

The reaction according to the invention can be expressed as follows:

$$Ar-(NO_2)_x + x(3CO) + xR-OH \xrightarrow{cat.} Ar-(NHCOOR)_x + x(2CO_2),$$

wherein Ar is an aryl or heteroaryl radical, R is a residue of an alcohol and x equals 1, 2 or 3.

Processes of catalytic carbonylation of aromatic nitroderivatives by means of carbon monoxide and an alcohol already known.

According to the process described in U.S.Patent No. 3,993,685, use is made of a catalytic system consisting of a noble metal (Pd) or its salts and of a tertiary amine, for instance pyridine, in a considerable excess from 5 to 10 moles per mole of aromatic nitroderivative.

The use of large amounts of tertiary amine gives rise, however, to numerous technical and economic drawbacks, concerning the recovery of the products and the recycle of the catalytic system, especially on an industrial scale.

According to another process described in U.S. Patent No. 4,186,269, use is made of considerable amounts, compared with the starting nitroderivative, of a catalytic system consisting of:

1) a noble metal such as Pd, Rh or Ru or a salt thereof;

2) a Lewis acid such as $FeCl_3$, or $SnCl_4$; and

3) a tertiary amine such as triethylamine, or pyridine.

The system consisting of palladium supported on carbon or alumina, anhydrous $FeCl_3$ and pyridine, in molar ratios of pyridine/Pd = 130, pyridine/starting nitroderivative = 0.46, $FeCl_3$/Pd = 40, proved to be particularly effective.

The tertiary amine, employed in a considerable excess with respect to the metal, is generally used for increasing the catalytic activity of the system Pd/Lewis acid and for minimizing the corrosion of the synthesis reactor, due to the presence of the Lewis acid. However, the use of large amounts of tertiary amine and of Lewis acid presents considerable problems, relating to the filtration, to the products and catalyst recovery and, in general, to the handling of the reactants, with technical and economic drawbacks, particularly when carrying out the process on an industrial scale.

The present invention aims to provide a process for preparing aromatic urethanes with high yields and selectivities through a simple and economic process, which does not give rise to corrosion phenomena of the reactor, which can be carried out industrially with ease, and which does not generally present the drawbacks of the processes of the prior art.

The present invention provides a process for preparing an aromatic urethane by reacting a nitroaromatic compound with carbon monoxide and an alcohol in the presence of a catalyst, characterized in that a nitroaromatic compound of formula $Ar(NO_2)_x$ is reacted with carbon monoxide and an alcohol at a temperature of from 25°C to 250°C and under a pressure of from 1 to 150 atm, in the presence of a catalytic system consisting of 1) a noble metal supported on an inert carrier and of an aromatic bidentate nitrogenous chelating agent (Chel), or of a salt of a noble metal $(MY_2)$ and of said chelating agent, which can be optionally combined as a complex of formula $M(Chel)Y_2$, and optionally of 2) a Brönsted acid, wherein Ar is an aryl or heteroaryl radical, optionally substituted with one or more groups inert under under the reaction conditions, x equals 1, 2 or 3, M is a noble metal, and

4

Y is an organic or inorganic anion.

In the process according to the invention the reaction between the nitroaromatic compound, carbon monoxide and alcohol is carried out in the presence of small amounts of a very active and selective catalytic system, consisting of a supported noble metal or a salt thereof, of an aromatic bidentate nitrogenous chelating agent and, optionally, of a Brönsted acid, using very low molar ratios of chelating agent/metal and chelating agent/nitroaromatic compound.

The catalyst is employed in catalytic amounts, and preferably there are used from $1.10^{-1}$ to $1.10^{-4}$ grams atoms of noble metal contained in the catalyst per mole of nitroaromatic compound. Also, advantageous results are obtained by using molar ratios of Chel/M of from $1.10^{-1}$ to 10 and Chel/Ar$(NO_2)_x$ of from $1.10^{-1}$ to $1.10^{-3}$.

The noble metal, preferably Pd, Pt, Rh, Ru, more preferably palladium or platinum, is employed in the form supported on an inert carrier, especially on a porous inert carrier, or as a $MY_2$ salt or as a complex having formula $M(Chel)Y_2$. Finely divided carbon, silica and alumina can be suitably used as inert carriers; finely divided carbon is particularly preferred.

Acetates, trifluoroacetates, mesitoates, chlorides and bromides, but preferably acetates, can be used as

5

anions Y of the metal salt.

The **aromatic bidentate nitrogenous chelating agent** is preferably selected from 1,10-phenanthroline(phen) and 2,2'-dipyridyl and their substituted derivatives such as 4,7-diphenyl-1,10-phenanthroline, 3,4,7,8-tetramethyl-1,10-phenanthroline (TM phen) and 4,4'-dimethyl-2,2'-dipyridyl.

The **complexes of formula** $M(Chel)Y_2$ are prepared according to known techniques.

A good catalytic activity and selectivity can be obtained in the absence of a Brönsted acid. It is preferred, however, in order to obtain a higher activity and selectivity, to use a Bronsted acid in an amount generally not greater than 100 moles, more preferably from 1 to 10 moles, per gram atom of noble metal.

The Bronsted acid can be suitably selected from mesitoic acid, acetic acid, 2,4,6-trimethylbenzenesulfonic acid, 2,6-dimethylbenzoic acid, carbonic acid and hydrochloric acid, but it is preferably selected from acids which are not esterificable or are esterificable only with difficulty, such as mesitoic acid.

Examples of catalysts which can be used in the process according to the invention are:

Pd/C + TM phen + 8 mesitoic acid

Pd/C + 0.5 TM phen + 8 mesitoic acid

6

Pd/C + TM phen + 4 mesitoic acid

Pd/C + 0.5 TM phen

Pd/C + 3 phen + 8 mesitoic acid

Pd/C + 3 phen + 100 acetic acid

Pd (acetate)$_2$ + 3 phen + 8 mesitoic acid

Pd TM phen (acetate)$_2$ + 8 mesitoic acid

Pd TM phen (acetate)$_2$ + TM phen + 10 mesitoic acid, wherein "Pd/C" stands for palladium supported on carbon,
"phen" stands for 1,10-phenanthroline, "TM phen" stands
for 3,4,7,8-tetramethyl-1,10-phenanthroline, and the
numbers indicate the moles of components present for
each gram atom of noble metal.

It will be apparent that the catalyst used in the
process of the invention may consist of an aromatic
bidentate nitrogenous chelating agent in addition to a
said chelating agent and a noble metal salt combined as
a said complex of formula M(Chel)Y$_2$, and optionally of
a Brönsted acid.

Complex Pd TM phen (acetate)$_2$ is prepared by
simple mixing between palladium acetate and
3,4,7,8-tetramethyl-1,10 phenanthroline in an anhydrous
alcoholic medium.

The alcohol is suitably selected from aliphatic,
cycloaliphatic or aromatic, mono-or polyhydroxylated
alcohols or mixtures thereof, which can optionally
contain substituents containing oxygen or one or more halogen atoms. Among the aliphatic and cycloaliphatic alcohols use may be made of: methyl, ethyl, n-propyl, iso-propyl, n-butyl, iso-butyl, terbutyl and cyclohexyl alcohols, $CF_3-CH_2-OH$, ethylene glycol, propylene glycol and glycerol, and among the aromatic alcohols use is preferably made of phenol.

Among the alcohols, methyl and ethyl alcohols are preferred, as the urethanes obtained through these alcohols can be decomposed thermically to isocyanates with ease.

The alcohol may be employed in stoichiometic amounts with respect to the nitrogroups present in the nitroaromatic compound, but it is preferred to employ the alcohol in a small excess since it acts as a solvent as well. An inert organic solvent may also be used, in combination with alcohol.

The nitroaromatic compound may be suitably nitrobenzene, nitrotoluene, nitronaphthalene, dinitrobenzene, dinitrotoluene, a nitropyridine or a nitroquinoline.

Advantageous results are obtained by operating at a temperature of from 100°C to 190°C and under a carbon monoxide pressure of from 10 to 60 atm.

At the end of the reaction, the product is separated according to conventional techniques. For instance, the reaction mixture may be filtered in order to remove

8

any metal or supported metal possibly present, after which there is carried out a distillation in order to remove the alcohol in excess up to urethane precipitation.

The catalyst present in the solution, after urethane separation, can be removed from the reaction medium by adsorption, for instance on bone charcoal, silica or alumina.

The Brönsted acid, when present, can be separated by conventional techniques, for instance mesitoic acid can be separated by treating the solution with an aqueous solution of NaOH, while acetic acid can be removed as an ester by distillation together with the alcohol.

The process according to the invention may be suitably carried out as follows.

Into a thermoregulated reactor, provided with reactant-feeding systems and a stirrer, there are introduced, under a carbon monoxide atmosphere, the alcoholic solution containing the nitroaromatic compound, then the catalyst and optionally the Brönsted acid. There are then introduced the desired amount of carbon monoxide under pressure and the reaction is carried out at the established temperature and for the established time. At the end of the reaction, controlled for instance by gaschromatography, the product is recovered according to conventional techniques.

The obtained products consist of of arylurethanes and can be generally used as active intermediates for organic synthesis in the field of fine chemistry, for instance for the preparation of substances useful in agriculture. Among them, phenyl urethane and 2,4-toluenediurethane are employed mainly in the synthesis of isocyanates and therefrom polyurethanes.

By the process according to the present invention, because of the high activity and selectivity of the employed catalysts, arylurethanes can be obtained with high conversions, higher than 99%, in short times and under conditions of comparatively modest pressures.

The invention will be further described with reference to the following illustrative Examples.
The symbols used in the Examples are:
Pd/C = palladium supported on carbon;
phen = 1,10-phenanthroline;
3,4,7,8 $Me_4$phen =
3,4,7,8-tetramethyl-1,10-phenanthroline;
2,4,6 TMBA = 2,4,6-trimethylbenzoic or mesitoic acid;
Sub = substrate or nitroaromatic compound;
Chel = chelating agent as defined in the disclosure.

EXAMPLE 1

1.23 g of nitrobenzene ($1.10^{-2}$ moles) were added to 8 ml of dehydrated ethanol. The solution thus obtained was conveyed, under a CO flow, into a 100 ml autoclave, containing 170.2 mg of Pd/C at 5% of Pd ($8.10^{-5}$ moles of Pd), 18.90 mg of 3,4,7,8 $Me_4$phen ($8.10^{-5}$ moles) and 105.1 mg of 2,4,6-trimethylbenzoic acid (2,4,6 TMBA) ($6.4.10^{-4}$ moles).

Then the autoclave was loaded with carbon monoxide at a CO pressure of 40 bar at room temperature and then it was heated at 180°C, under magnetic stirring.

After 2 hours from start of heating, the autoclave was cooled, the contents filtered in order to remove Pd/C, and the solution was analysed by gaschromatography.

A conversion of 99% was obtained, with a yield of ethyl phenylurethane of 95.5%; the selectivity of urethane with respect to the reacted nitrobenzene was 96.5%.

Ratios: Sub/Pd = 125; Chel/Pd = 1; 2,4,6 TMBA/Pd = 8.

EXAMPE 2

2.46 g of nitrobenzene ($2.10^{-2}$ moles) were added to 8 ml of dehydrated ethanol. The solution thus obtained was conveyed, under a CO flow, into a 100 ml autoclave, containing 170.2 mg of Pd/C at 5% of Pd ($8.10^{-5}$ moles of Pd), 37.81 mg of 3,4,7,8 $Me_4$phen ($1.6 \times 10^{-4}$ moles) and 131.3 mg of 2,4,6-trimethylbenzoic acid ($8.10^{-4}$ moles).

Then the autoclave was loaded with 40 bar of carbon monoxide at room temperature and then it was heated at

11

180°C, under magnetic stirring.

After 2 hours from the start of heating, the autoclave was cooled, the contents filtered in order to remove Pd/C, and the solution was analysed by gaschromatography.

A conversion of 97% was obtained, with a yield of ethyl phenylurethane of 92.5%; the selectivity of urethane with respect to the reacted nitrobenzene was 95.5%.

Ratios: Sub/Pd = 250; chel/Pd = 2; 2,4,6 TMBA/Pd = 10.

EXAMPLE 3 (for comparison)

There was followed the procedure as described in Example 1, without, however, adding 3,4,7,8 $Me_4$phen. After 2 hours from the start of heating there was obtained again the starting nitrobenzene, practically unaltered.

Ratios: Sub/Pd = 125; 2,4,6 TMBA/Pd = 8.

EXAMPLE 4

There was followed the procedure as described in Example 1, using, however, 8 ml of dehydrated methanol instead of ethanol. After 2 hours from the start of heating a conversion of 99.5% was obtained, with a yield of methyl phenylurethane of 95.5%; the selectivity of urethane with respect to the reacted nitrobenzene was 96%.

Ratios: Sub/Pd = 125; chel/Pd = 1; 2,4,6 TMBA/Pd = 8.

EXAMPLE 5

There was followed the procedure as described in Example 1, using, however, 9.45 mg of 3,4,7,8 Me$_4$phen (4.10$^{-5}$ moles). After 2 hours from the start of heating a conversion of 77% was obtained, with a yield of ethyl phenylurethane of 74.5%; the selectivity of urethane with respect to the reacted nitrobenzene was 97%.

Ratios: Sub/Pd = 125; chel/Pd = 0.5; TMBA/Pd = 8.

EXAMPLE 6

There was followed the procedure as described in Example 1, using, however, 4 ml of dehydrated ethanol. After 2 hours from the start of heating a conversion of 98.5% was obtained, with a yield of ethyl phenylurethane of 95.5%; the selectivity of urethane with respect to the reacted nitrobenzene was 97%.

Ratios: Sub/Pd = 125; chel/Pd = 1; 2,4,6 TMBA/Pd = 8.

EXAMPLE 7

There was followed the procedure as described in Example 1, using, however, 52.55 mg of 2,4,6-trimethylbenzoic acid (3.2 x 10$^{-4}$ moles). After 2 hours from the start of heating a conversion of 98% was obtained, with a yield of ethyl phenylurethane of 94%; the selectivity of urethane with respect to the reacted nitrobenzene was 96%.

Ratios: Sub/Pd = 125; chel/Pd = 1; 2,4,6 TMBA/Pd = 4.

EXAMPLE 8

There was followed the procedure as described in Example 1, using, however, 26.26 mg of 2,4,6-trimethylbenzoic acid ($1.6 \times 10^{-4}$ moles). After 2 hours from the start of heating a conversion of 91% was obtained, with a yield of ethyl phenylurethane of 85.5%; the selectivity of urethane with respect to the reacted nitrobenzene was 94%.

Ratios: Sub/Pd = 125; chel/Pd = 1; 2,4,6 TMBA/Pd = 2.

EXAMPLE 9

There was followed the procedure as described in Example 1, using, however, 13.13 g of 2,4,6-trimethylbenzoic acid ($8.10^{-5}$ moles). After 2 hours from the start of heating a conversion of 72.5% was obtained, with a yield of ethyl phenylurethane of 58%; the selectivity of urethane with respect to the reacted nitrobenzene was 80%.

Ratios: Sub/Pd = 125; chel/Pd = 1; 2,4,6 TMBA/Pd = 1.

EXAMPLE 10

There was followed the procedure as described in Example 1, without, however, adding 2,4,6-trimethylbenzoic acid. After 2 hours from the start of heating a conversion of 51.5% was obtained, with a yield of ethyl phenylurethane of 25.5%; the selectivity of urethane with respect to the reacted nitrobenzene was 49.5%.

Ratios: Sub/Pd = 125; chel/Pd = 1.

14

## EXAMPLE 11

There was followed the procedure as described in Example 10, using, however, 9.45 mg of 3,4,7,8 $Me_4$phen ($4.10^{-5}$ moles). After 2 hours from the start of heating a conversion of 25% was obtained, with a yield of ethyl phenylurethane of 19%; the selectivity of urethane with respect to the reacted nitrobenzene was 76%.

Ratios: Sub/Pd = 125; chel/Pd = 0.5.

## EXAMPLE 12

There was followed the procedure as described in Example 10, using, however, 4.73 mg of 3,4,7,8 $Me_4$phen ($2.10^{-5}$ moles). After 2 hours from the start of heating a conversion of 16.5% was obtained, with a yield of ethyl phenylurethane of 13%; the selectivity of urethane with respect to the reacted nitrobenzene was 80%.

Ratios: Sub/Pd = 125; chel/Pd = 0.25.

## EXAMPLE 13

There was followed the procedure as described in Example 11, using, however, 8 ml of dehydrated methanol. After 2 hours from the start of heating a conversion of 28.5% was obtained, with a yield of methyl phenylurethane of 22.5%; the selectivity of urethane with respect to the reacted nitrobenzene was 79%.

Ratios: Sub/Pd = 125; chel/Pd = 0.5.

EXAMPLE 14

There was followed the procedure as described in Example 1, using, however, 47.58 mg of phen. $H_2O$ (2.4 x $10^{-4}$ moles). After 2 hours from the start of heating a conversion of 100% was obtained, with a yield of ethyl phenylurethane of 93.5%; the selectivity of urethane with respect to the reacted nitrobenzene was 93.5%.

Ratios: Sub/Pd = 125; chel/Pd = 3; 2,4,6 TMBA/Pd = 8.

EXAMPLE 15

There was followed the procedure as described in Example 14, using, however, 43.25 mg of anhydrous 1,10-phenanthroline. After 2 hours from the start of heating a conversion of 100% was obtained, with a yield of ethyl phenylurethane of 95%; the selectivity of urethane with respect to the reacted nitrobenzene was 95%.

Ratios: Sub/Pd = 125; chel/Pd = 3; 2,4,6 TMBA/Pd = 8.

EXAMPLE 16

1.23 g of nitrobenzene ($1.10^{-2}$ moles) and o.5 g of glacial acetic acid ($8.10^{-3}$ moles) were added to 8 ml of dehydrated ethanol.

The solution thus obtained was conveyed, under a CO flow, into a 100 ml autoclave, containing 170.2 mg of Pd/C 5% ($8.10^{-5}$ moles of Pd) and 47.58 mg of phen.$H_2O$ (2.4 x $10^{-4}$ moles).

Then the autoclave was loaded with 40 bar of carbon

16

monoxide at room temperature and then it was heated at 180°C.

After 2 hours from the start of heating the autoclave was cooled, the contents filtered in order to remove Pd/C, and the solution was analysed by gaschromatography.

A conversion of 100% was obtained, with a yield of ethyl phenylurethane of 90%; the selectivity of urethane with respect to the reacted nitrobenzene was 90%.

Ratios: Sub/Pd = 125; chel/Pd = 3; $CH_3COCl/Pd$ = 100.

EXAMPLE 17 (for comparison)

There was followed the procedure as described in Example 16, without, however, adding phenanthroline. After 2 hours from the start of heating there was obtained again the starting nitrobenzene, practically unaltered.

Ratios: Sub/Pd = 125; $CH_3COOH/Pd$ = 100.

EXAMPLE 18

There was followed the procedure as described in Example 16, using, however, 8 ml of bottle absolute alcohol (RPE). After 2 hours from the start of heating a conversion of 100% was obtained, with a yield of ethyl phenylurethane of 88.5%; the selectivity of urethane with respect to the reacted nitrobenzene was 88.5%.

Ratios: Sub/Pd = 125; chel/Pd = 3; $CH_3COOH/Pd$ = 100.

EXAMPLE 19

There was followed the procedure as described in Example 16, using, however, 18.90 mg of 3,4,7,8 $Me_4phen$ ($8.10^{-5}$ moles). After 2 hours from the start of heating a conversion of 99.5% was obtained, with a yield of ethyl phenylurethane of 90%; the selectivity of urethane with respect to the reacted nitrobenzene was 90.5%.

Ratios: Sub/Pd = 125; chel/Pd = 1; $CH_3COOH/Pd$ = 100.

EXAMPLE 20

There was followed the procedure as described in Example 16, using, however, as substrate 1.37 g of p-nitrotoluene ($1.10^{-2}$ moles), which were fed directly into the autoclave. After 2 hours after the start of heating a p-nitrotoluene conversion of 100% was obtained, with a yield of the corresponding ethyl urethane of 92%; the selectivity of urethane with respect to the reacted p-nitrotoluene was 92%.

Ratios: Sub/Pd = 125; chel/Pd = 3; $CH_3COOH/Pd$ = 100.

EXAMPLE 21

There was followed the procedure as described in Example 16, using, however, 20 µl of HCl 37% ($2.4 \times 10^{-4}$ moles) instead of glacial acetic acid. After 2 hours from the start of heating a conversion of 57% was obtained, with a yield of ethyl phenylurethane of 44%; the selectivity of urethane with respect to the reacted nitrobenzene was 77%.

Ratios: Sub/Pd = 125; chel/Pd = 3; HCl/Pd = 3.

EXAMPLE 22 (for comparison)

There was followed the procedure as described in Example 21, without, however, adding phenanthroline. After 2 hours from the start of heating there was obtained again the starting nitrobenzene, practically unaltered.

Ratios: Sub/Pd = 125; HCl/Pd = 3.

EXAMPLE 23

1.23 g of nitrobenzene ($1.10^{-2}$ moles) were added to 8 ml of dehydrated ethanol.

The solution thus obtained was conveyed, under a CO flow, into a 100 ml autoclave, containing 17.96 mg of Pd $(CH_3COO)_2$ ($8.10^{-5}$ moles), 47.58 mg of phen.$H_2O$ ($2.4 \times 10^{-4}$ moles) and 105.1 mg of 2,4,6-trimethylbenzoic acid ($6.4 \times 10^{-4}$ moles).

Then the autoclave was loaded with 40 bar of carbon monoxide at room temperature and then it was heated at 180°C, under magnetic stirring.

After 2 hours from the start of heating, the autoclave was cooled, the contents filtered in order to remove traces of formed metal Pd, and the solution was analysed by gaschromatography.

A conversion of 100% was obtained, with a yield of ethyl phenylurethane of 93.5%; the selectivity of urethane with respect to the reacted nitrobenzene was 93.5%.

Ratios: Sub/Pd = 125; chel/Pd = 3; 2,4,6 TMBA/Pd = 8.

EXAMPLE 24

There was followed the procedure as described in Example 23, using, however, 8 ml of dehydrated methanol instead of ethanol After 2 hours from the start of heating a conversion of 100% was obtained, with a yield of methyl phenylurethane of 93.5%; the selectivity of urethane with respect to the reacted nitrobenzene was 93.5%.

Ratios: Sub/Pd = 125, chel/Pd = 3; 2,4,6 TMBA/Pd = 8.

EXAMPLE 25

1.23 g of nitrobenzene ($1.10^{-2}$ moles) were added to 8 ml of dehydrated ethanol.

The solution thus obtained was conveyed, under a CO flow, into a 100 ml autoclave containing 32.37 mg of complex [Pd(phen)(CH$_3$COO)$_2$] ($8.10^{-5}$ moles) and 105.1 mg of 2,4,6-trimethylbenzoic acid ($6.4 \times 10^{-4}$ moles).

Then the autoclave was loaded with 40 bar of carbon monoxide at room temperature and then it was heated at 180°C, under magnetic stirring.

After 2 hours from the start of heating the autoclave was cooled, the contents filtered in order to remove traces of formed metal Pd, and the solution was analysed by gaschromatography.

A conversion of 82% was obtained, with a yield of ethyl phenylurethane of 77.5%, the selectivity of urethane with respect to the reacted nitrobenzene was 94.5%.

Ratios: Sub/Pd = 125; chel/Pd = 1; 2,4,6 TMBA/Pd = 8.

EXAMPLE 26

1.23 g of nitrobenzene ($1.10^{-2}$ moles) were added to 8 ml of dehydrated ethanol.

The solution thus obtained was conveyed, under a CO flow, into a 100 ml autoclave, containing 32.37 mg of complex [Pd phen $(CH_3COO)_2$] ($8.10^{-5}$ moles), 14.42 mg of anhydrous phenanthroline ($8.10^{-5}$ moles) and 105.1 mg of 2,4,6-trimethylbenzoic acid ($6.4 \times 10^{-4}$ moles).

Then the autoclave was loaded with 40 bar of carbon monoxide and it was heated at 140°C under magnetic stirring.

After 2 hours from the start of heating the autoclave was cooled, the solution contained therein filtered in order to remove possible traces of metal Pd (absent in this case), and then analysed by gaschromatography.

A conversion of 96.5% was obtained, with a yield of ethyl phenylurethane of 92%; the selectivity of urethane with respect to the reacted nitrobenzene was 95.5%.

Ratios: Sub/Pd = 125; chel/Pd = 2; 2,4,6 TMBA/Pd = 8.

EXAMPLE 27

There was followed the procedure as described in Example 26, heating, however, the autoclave at 120°C. After 6 hours from the start of heating a conversion of 98.5% was obtained, with a yield of ethyl phenylurethane of 91.5%; the selectivity of urethane with respect to the reacted nitrobenzene was 93%.

Ratios: Sub/Pd = 125; chel/Pd = 2; 2,4,6 TMBA/Pd = 8.

EXAMPLE 28

There was followed the procedure as described in Example 25, using, however, 44.55 mg of complex [Pd(4,7-diphenyl-phen)(CH$_3$COO)$_2$] ($8.10^{-5}$ moles). After 2 hours from the start of heating a conversion of 100% was obtained, with a yield of ethyl phenylurethane of 95%; the selectivity of urethane with respect to the reacted nitrobenzene was 95%.

Ratios: Sub/Pd = 125; chel/Pd = 1; 2,4,6 TMBA/Pd = 8.

EXAMPLE 29

Complex [Pd(3,4,7,8 Me$_4$phen)(CH$_3$COO)$_2$] was prepared, by adding 450 mg of 3,4,7,8 Me$_4$phen ($1.9 \times 10^{-3}$ moles) to 400 mg of Pd(CH$_3$COO)$_2$ ($1.8 \times 10^{-3}$ moles) suspended in 25 ml of absolute ethanol.

The solution turned limpid, changing from bright orange to yellow and within 1 hour a light yellow microcrystalline solid precipitated.

After filtration the solid was washed with absolute

22

ethanol and dried under vacuum at room temperature.

The solid obtained with a yield of 90% gave upon elemental analysis the following results:

|  | % C | % H | % N |
|---|---|---|---|
| Calculated | 52.1 | 4.81 | 6.08 |
| Found | 51.9 | 4.87 | 5.99 |

There was then followed the procedure as described in Example 25, using, however, 36.86 mg of complex [Pd(3,4,7,8 Me$_4$ phen) (CH$_3$COO)$_2$] ($8.10^{-5}$ moles) thus prepared.

After 1 hour from the start of heating a conversion of 95.5% was obtained, with a yield of ethyl phenylurethane of 92%; the selectivity of urethane with respect to the reacted nitrobenzene was 96.5%.

Ratios: Sub/Pd = 125; chel/Pd = 1; 2,4,6 TMBA/Pd = 8.

EXAMPLE 30

There was followed the procedure as described in Example 29, adding 9.45 mg of 3,4,7,8 Me$_4$ phen ($4.10^{-5}$ moles), which were fed directly into the autoclave. After 1 hour from the start of heating a conversion of 99% was obtained, with a yield of ethyl phenylurethane of 93.5%; the selectivity of urethane with respect to the reacted nitrobenzene was 94.5%.

Ratios: Sub/Pd = 125; chel/Pd = 1.5; 2,4,6 TMBA/Pd = 8.

EXAMPLE 31

There was followed the procedure as described in Example 29, adding 18.90 mg of 3,4,7,8 Me$_4$ phen (8.10$^{-5}$ moles). After 1 hour from the start of heating a conversion of 100% was obtained, with a yield of ethyl phenylurethane of 94.5%; the selectivity of urethane with respect to the reacted nitrobenzene was 94.5%.

Ratios: Sub/Pd = 125; chel/Pd = 2; 2,4,6 TMBA/Pd = 8.

EXAMPLE 32

There was followed the procedure as described in Example 31, heating, however, the autoclave at 160°C. After 1 hour from the start of heating, the autoclave was cooled, the solution contained therein was filtered in order to remove possible traces of metal Pd (absent in this case), and analysed by gaschromatography. A conversion of 86% was obtained, with a yield of ethyl phenylurethane of 81.5%; the selectivity of urethane with respect to the reacted nitrobenzene was 94.5%.

Ratios: Sub/Pd = 125; chel/Pd = 2; 2,4,6 TMBA/Pd = 8.

EXAMPLE 33

There was followed the procedure as described in Example 31, heating, however, the autoclave at 140°C. After 2 hours from the start of heating a conversion of 56.5% was obtained, with a yield of ethyl phenylurethane of 53.5%; the selectivity of urethane with respect to the reacted nitrobenzene was 94.7%.

Ratios: Sub/Pd = 125; chel/Pd = 2; 2,4,6 TMBA/Pd = 8.

## EXAMPLE 34

There was followed the procedure as described in Example 31, doubling, however, the substrate amount, i.e., addding in the solution 2.46 g of nitrobenzene ($2.10^{-2}$ moles). After 2 hours from the start of heating a conversion of 100% was obtained, with a yield of ethyl phenylurethane of 96%; the selectivity of urethane with respect to the reacted nitrobenzene was 96%.

Ratios: Sub/Pd = 250; chel/Pd = 2; 2,4,6 TMBA/Pd = 8.

## EXAMPLE 35

There was followed the procedure as described in Example 1, using, however, 321.14 mg of Pt/C 5% ($8.10^{-5}$ moles of Pt) instead of Pd/C. After 2 hours from the start of heating a conversion of 10% was obtained, with a yield of ethyl phenylurethane of 6%; the selectivity of urethane with respect to the reacted nitrobenzene was 60%.

Ratios: Sub/Pt = 125; chel/Pt = 1; 2,4,6 TMBA/Pt = 8.

## EXAMPLE 36

8 ml of dehydrated ethanol were conveyed, under a CO flow, into a 100 ml autoclave containing 0.91 g of 2,4-dinitrotoluene ($5.10^{-3}$ moles), 36.86 mg of complex [$Pd(3,4,7,8\ Me_4\ phen)(CH_3COO)_2$] ($8.10^{-5}$ moles) and 105.1 mg of 2,4,6-trimethylbenzoic acid (6.4 x $10^{-4}$ moles).

Then the autoclave was loaded with 40 bar of carbon monoxide at room temperature and then it was heated at 180°C, under magnetic stirring.

After 1 hour from the start of heating the autoclave was cooled, the contents filtered under heat in order to remove the traces of formed metal Pd, and the solution was analysed by gaschromatography.

At room temperature, the ethyl 2,4-toluene-diurethane separated only partly as a solid from the solution; an aqueous solution of KOH was added, in order to obtain a complete precipitation.

The separate solid consisted substantially of ethyl 2,4-toluene-diurethane, whereas the salified 2,4,6-trimethylbenzoic acid remained in the alkaline solution, from which it could be recovered by addition of HCl.

Gaschromatographic analyses of the solution and infra red spectra of the isolated solid showed that the 2,4-dinitrotoluene had completely disappeared and that the solid was a mixture of mono- and di-urethane, with a yield of diurethane with respect to the reacted dinitrotoluene of at least 90%.

Ratios: Sub/Pd = 62.5; chel/Pd = 1; 2,4,6 TMBA/Pd = 8.

EXAMPLE 37

There was followed the procedure as described in Example 36, using, however, 1.82 mg of

26

2,4-dinitrotoluene ($1.10^{-2}$ moles). After 2 hours from the start of heating, gaschromatographic analyses and infra red spectra showed that the conversion was 100% and that, in the isolated solid, the yield of ethyl diurethane was at least 90% with respect to the reacted dinitrotoluene.

Ratios: Sub/Pd = 125; chel/Pd = 1; 2,4,6 TMBA/Pd = 8.

EXAMPLE 38

There was followed the procedure as described in Example 36, using, however, as catalyst 170.2 mg of Pd/C 5% ($8.10^{-5}$ moles of Pd) and 18.90 mg of 3,4,7,8 $Me_4$ phen ($8.10^{-5}$ moles), which were fed directly into the autoclave. After 2 hours from the start of heating, gaschromatographic analyses and infra red spectra showed that the conversion was 100% and that, in the isolated solid, the yield of ethyl diurethane was at least 85% with respect to the reacted dinitrotoluene.

Ratios: Sub/Pd = 62.5; chel/Pd = 1; 2,4,6 TMBA/Pd = 8.

EXAMPLE 39

There was followed the procedure as described in Example 37, using, however, 8 ml of dehydrated methanol. In this case, the precipitation of methyl 2,4-toluene-diurethane from the solution, that had been extracted still hot from the autoclave, was practically complete, already at room temperature.

After 2 hours from the start of heating, gaschromatographic analyses and infra red spectra showed that the conversion was 100% and that, in the isolated solid, the yield of methyl diurethane was at least 90% with respect to the reacted dinitrotoluene.

Ratios: Sub/Pd = 125; chel/Pd = 1; 2,4,6 TMBA/Pd = 8.

EXAMPLE 40

There was followed the procedure as described in Example 39, using, however, 0.91 g of 2,4-dinitrotoluene ($5.10^{-3}$ moles). After 1.5 hour from the start of heating, gaschromatographic analyses and infra red spectra showed that the conversion was 100% and that, in the isolated solid, the yield of methyl diurethane was at least 90% with respect to the reacted dinitrotoluene. Ratios: Sub/Pd = 125; chel/Pd = 1; 2,4,6 TMBA/Pd = 8.

EXAMPLE 41 (for comparison)

1 g of nitrobenzene ($8.1 \times 10^{-3}$ moles) and 0.3 g of pyridine ($3.8 \times 10^{-3}$ moles) were added to 25 ml of dehydrated ethanol.

The solution thus obtained was conveyed, under a CO flow, into a 100 ml autoclave, containing 62.5 mg of Pd/C 5% ($2.9 \times 10^{-5}$ moles of Pd) and 0.2 g of anhydrous $FeCl_3$ ($1.2 \times 10^{-3}$ moles).

Then the autoclave was loaded with 50 bar of carbon monoxide at room temperature and then it was heated at 180°C, under magnetic stirring.

After 2 hours from the start of heating, the autoclave was cooled, the contents filtered, and the solution was analysed by gaschromatography.

A conversion of 40% was obtained, with a yield of ethyl phenylurethane of 37.5%; the selectivity in urethane with respect to the reacted nitrobenzene was 94%.

Ratios: Sub/Pd = 280; Fe/Pd = 40; Pyridine/Pd = 130.

EXAMPLE 42 (for comparison)

1.23 g of nitrobenzene ($1.10^{-2}$ moles) and 1.89 g of pyridine ($2.4 \times 10^{-2}$ moles) were added to 8 ml of dehydrated ethanol.

The solution thus obtained was conveyed, under a CO flow, into a 100 ml autoclave, containing 170.2 mg of Pd/C 5% ($8.10^{-5}$ moles of Pd).

Then the autoclave was loaded with 40 bar of carbon monoxide at room temperature and then it was heated at 180°C, under magnetic stirring.

After 2 hours from the start of heating the autoclave was cooled, the contents filtered in order to remove Pd/C, and the solution was analysed by gaschromatography.

A conversion of 1.5% was obtained, with a yield of ethyl phenylurethane that was practically nil (traces).

Ratios: Sub/Pd = 125; Pyridine/Pd = 300.

EXAMPLE 43 (for comparison)

There was followed the procedure as described in Example 42, using, however, 14.18 mg of $PdCl_2$

($8.10^{-5}$ moles) instead of Pd/C. After 2 hours from the start of heating a conversion of 14.5% was obtained, with a yield of ethyl phenylurethane of 12.5%; the selectivity of urethane with respect to the reacted nitrobenzene was 86%.

Ratios: Sub/Pd = 125; Pyridine/Pd = 300.

CLAIMS

1. A process for preparing an aromatic urethane by reacting a nitroaromatic compound with carbon monoxide and an alcohol in the presence of a catalyst, characterized in that a nitroaromatic compound of formula $Ar(NO_2)_x$ is reacted with carbon monoxide and an alcohol at a temperature of from 25 to 250°C and under a pressure of from 1 to 150 atm, in the presence of a catalyst consisting of (1) a noble metal supported on an inert carrier and of an aromatic bidentate nitrogenous chelating agent (Chel), or of a salt of a noble metal $(MY_2)$ and of said chelating agent, optionally combined as a complex of formula $M(Chel)Y_2$, and optionally (2) of a Brönsted acid, wherein: Ar is an aryl or heteroaryl radical, optionally substituted with one or more groups inert under the reaction conditions, x equals 1, 2 or 3, M is a noble metal, and Y is an organic or inorganic anion.

2. A process as claimed in claim 1, characterized in that the molar ratio between the noble metal contained in the catalyst and the nitroaromatic compound is from $1.10^{-1}$ to $1.10^{-4}$, the molar ratio between the chelating agent and the metal is from $1.10^{-1}$ to 10, and the molar ratio between the chelating agent and the nitrogenous aromatic compound is from $1.10^{-1}$ to

$1.10^{-3}$.

3. A process as claimed in claim 1 or 2, characterized in that the noble metal is selected from palladium and platinum and in that the inert carrier is finely divided carbon.

4. A process as claimed in claim 1 or 2, characterized in that the noble metal salt is an acetate, trifluoroacetate, mesitoate, chloride or bromide of palladium or platinum.

5. A process as claimed in any of claims 1 to 4, characterized in that the aromatic bidentate nitrogenous chelating agent is selected from 1,10-phenanthroline; 4,7-diphenyl-1,10-phenanthroline; 3,4,7,8-tetramethyl-1,10-phenanthroline; 2,2'-dipyridyl; and 4,4'-dimethyl-2,2'-dipyridyl.

6. A process as claimed in any of claims 1 to 5, characterized in that the Brönsted acid, when present, is selected from mesitoic acid, acetic acid, 2,4,6-trimethylbenzenesulfonic acid, 2,6-dimethylbenzoic acid, carbonic acid and hydrochloric acid, and is used in an amount not greater than 100 moles per gram atom of noble metal contained in the catalyst.

7. A process as claimed in claim 6, characterized in that the Brönsted acid is mesitoic acid used in an amount from 1 to 10 moles per gram atom of noble metal contained in the catalyst.

8. A process as claimed in any of claims 1 to 7, characterized in that the catalyst is selected from:

Pd/C + TM phen + 8 mesitoic acid

Pd/C + 0.5 TM phen + 8 mesitoic acid

Pd/C + TM phen + 4 mesitoic acid

Pd/C + 0.5 TM phen

Pd/C + 3 phen + 8 mesitoic acid

Pd/C + 3 phen + 100 acetic acid

Pd(acetate)$_2$ + 3 phen + 8 mesitoic acid

Pd TM phen (acetate)$_2$ + 8 mesitoic acid

Pd TM phen (acetate)$_2$ + TM phen + 10 mesitoic acid, wherein "Pd/C" stands for palladium supported on carbon, "phen" stands for 1,10-phenanthroline, "TM phen" stands for 3,4,7,8-tetramethyl-1,10-phenanthroline, and the numbers indicate the moles of components present for each gram atom of noble metal.

9. A process as claimed in any of claims 1 to 8, characterized in that the alcohol is selected from aliphatic, cycloaliphatic and aromatic, mono- or polyhydroxylated alcohols, which are optionally substituted with groups containing oxygen or with one or more halogen atoms.

10. A process as claimed in claim 9, characterized in that the alcohol is selected from methyl, ethyl, n-propyl, iso-propyl, n-butyl, isobutyl, tert. butyl and cyclohexyl alcohols, $CF_3-CH_2-OH$, ethyleneglycol,

propyleneglycol, glycerol and phenol.

11. A process as claimed in any of claims 1 to 10, characterized in that the nitroaromatic compound is selected from nitrobenzene, nitrotoluene, nitronaphthalene, dinitrobenzene, dinitrotoluene, nitropyridines, and nitroquinolines.

12. A process as claimed in any of claims 1 to 11, characaterized in that the reaction is carried out at a temperature of from 100 to 190°C and under a pressure of from 10 to 60 atm.